# EUROPEAN PATENT APPLICATION

(11) **EP 2 842 505 A1**
(43) Date of publication of application: **04.03.2015**
(21) Application number: 12874985.0
(22) Date of filing: 27.12.2012
(51) Int. Cl.: A61B 18/02

(54) **CYLINDRICAL PROBE OUTER CASING FOR CRYOSURGERY DEVICE, AND TREATMENT UNIT**

(30) Priority: 27.04.2012 JP 2012103156
(71) Applicant: DGS Computer Co., Ltd., Hachiouji-shi, Tokyo 192-0364 (JP)
(72) Inventor: IWATA, Kansei, Hachiouji-shi, Tokyo 192-0363 (JP)
(74) Representative: Epping - Hermann - Fischer
(86) International application number: PCT/JP2012/008359
(87) International publication number: WO 2013/160981

(57) **Abstract**

There are provided an external cylinder for a probe equipped in a cryosurgical apparatus and a therapeutic-device unit, which are able to protect normal cells near a lesion, provide higher heat efficiency for the freeze and thawing, and simplify the structure. The external cylinder for the probe equipped in the cryosurgical apparatus and the therapeutic-device unit which includes the external cylinder for the probe equipped in the cryosurgical apparatus are provided. The external cylinder includes a given range in which the freezing gas enables an ice ball to be formed on an outer circumference including the distal end portion; and adiabatic means arranged in a range other than the given range so as to prevent heat from being exchanged between the inner space and an outside.

## Description

### [Technical Field]

The present invention relates to an external cylinder for a probe equipped in a cryosurgical apparatus used for cryosurgery and a therapeutic-device unit including the external cylinder for the probe equipped in the cryosurgical apparatus.

### [Background Art]

It is known that cells of biological bodies can be necrosed by freezing the cells, and this feature of cells is used in cryosurgical apparatuses. In such a cryosurgical apparatus, only cells of a lesion are frozen and then thawed, during which thawing step a difference between salt concentrations is caused thereby destroying the inside of the cells so that the cells die. When it is defined that one cycle consists of the freezing and thawing steps, performing two to three cycles of the forgoing freezing and thawing steps makes it possible to necrose the cells of a lesion, such as a tumor (for example, refer to a non-patent literature 1).

The foregoing cryosurgical apparatus uses two types of high-pressure gases when the freeze and thawing are performed. In the high-pressure gasses, though depending on types of gas molecules, there is a gas whose temperature increases sharply and there is a gas whose temperature decreases sharply in response to rapidly expanding their volumes (which is known as Joule-Thompson effect). For instance, for decreasing the temperature, a high-pressure argon gas (whose temperature is approx. -160 degrees) is used, while for increasing the temperature, a helium gas (whose temperature is approx. +40 degrees) is used.

The foregoing cryosurgical apparatus uses as an external cylinder a hollow stainless pipe of a diameter of approx. 2 mm. A probe which emits the high-pressure gas from a fine pore of its distal end is inserted through this external cylinder for being charged therein. In this case, since the distal end of the external cylinder is made to puncture a lesion in advance, the cells of the lesion being targeted can be frozen to be necrosed in an appropriate manner.

By the way, the two sequences consisting of the freezing and thawing sequences are repeated through the distal end of the probe. In order to make effective use of energy, there is provided a heat exchanger (for example, refer to Patent Literature 1). Hence, in the probe, there are arranged an outward path through which the high-pressure gas is delivered toward the fine pore via the heat exchanger and a return path through which part of the gas emitted from the fine pore returns toward the base end of the probe via a space formed between the probe wall and the heat exchanger.

At the distal end, the freezing and thawing sequences are repeated by the foregoing heat exchanging mechanism, in which the distal end is exposed to an ultralow temperature during the freezing sequence. Hence it is necessary not to cause damage to normal cells around a lesion. Conventionally, there have been known a technique of providing the cylinder with a heat distribution characteristic necessary for an application of focused heat energy depending on where a lesion is located, which focused application is realized by applying intensity changes to directional characteristics of heat which has directionality. With this intention, a protrusion is arranged on a part of the circumferential edge of the cylinder distal end (for example, refer to Patent Literature 2).

### [Citation List]

### [Patent Literature]

[PTL 1] JP-B-4607813
[PTL 2] JP-B-4744284

### [NPL]

[NPL 1] Seishi Nakatsuka, Kansei Iwata et.al "On freeze-thaw sequence of vital organ of assuming the cryoablation for malignant lung tumors by using cryoprobe as heat source", CRYOBIOLOGY 61 (2010) 317-326

### [Summary of Invention]

### [Technical Problem]

However, the heat exchange process of the probe, which is performed in the external cylinder, causes the external cylinder to be cooled down. Due to this, on the outer surface of the external cylinder, frost formation or ice accretion is caused towards the base end thereof, which may damage normal cells around a lesion near the external cylinder.

In addition, in the case of the foregoing probe, the heat exchanger is arranged in the narrow space of the probe, which leads to problems of making the probe complex in its inner structure and raising manufacturing costs.

Moreover, due to the fact that the internal structure of the probe is made complex, the discharge path of the gas becomes narrower, thereby lowering a heat efficiency for the foregoing freeze and thawing. Hence, this may lead to cases in which Joule-Thompson effect is used insufficiently.

With consideration of the foregoing, an object of the present invention is to provide an external cylinder for a probe equipped in a cryosurgical apparatus used for cryosurgery and a therapeutic-device unit, which are able to protect normal cells near a lesion, provide higher heat efficiency for the freeze and thawing, and simplify the structure.

### [Solution to Problem]

In order to achieve the object, the external cylinder for a probe equipped in a cryosurgical apparatus according to the present invention is provided such that the external cylinder includes a hollow inner space in which the probe for the cryosurgical apparatus is loaded, the probe comprising a distal end portion which is able to puncture a lesion located inside an object, the probe emitting a freezing gas and a thawing gas alternately to each other, essentially characterized in that the external cylinder includes: a given range in which the freezing gas enables an ice ball to be formed on an outer circumference including the distal end portion; and adiabatic means arranged in a range other than the given range so as to prevent heat from being exchanged between the inner space and an outside.

According to the foregoing configuration, in the external cylinder for the probe equipped in the cryosurgical apparatus, there is provided a portion positionally corresponding to the range in which an ice ball is formed. The outer surface of the portion can be avoided from being lowered in temperature due to an adiabatic effect provided by the adiabatic means when the freezing gas is injected into a lesion during a treatment therefor.

Incidentally, the adiabatic means may have at least one of a closed space internally formed along a whole inner circumference of the external cylinder or a closed space formed to wholly cover an outer circumferential surface of the external cylinder. In addition, the closed space can be a vacuum layer and reflection means can be provided by applying specular finishing to a surface provided by the closed space. Alternatively, a vacuum layer can be formed in a whole circumference between the outer surface of a range other the given range and the inner space, and reflection means are arranged in the vacuum layer, the reflection means being composed of a barrier member. Still alternatively, the closed space can be loaded with an adiabatic material.

### [Advantageous Effects of Invention]

The external cylinder for the probe equipped in the cryosurgical apparatus is advantageous in that the external cylinder is structured with simplicity, has a higher efficiency for the freeze and thawing, and is able to protect normal cells which are present near a lesion.

### [Brief Description of Drawings]

[Fig. 1] Fig. 1 is a diagram showing a basic configuration of a cryosurgical apparatus.
[Fig. 2] Fig. 2 is a side sectional view showing a state in which a guide needle is inserted through an external cylinder equipped in a cryosurgical apparatus according to the present invention.
[Fig. 3] Fig. 3 is a side sectional view showing a state in which a probe is inserted through the external cylinder equipped in the cryosurgical apparatus according to the present invention.
[Fig. 4] Fig. 4 is a partial perspective view showing specular finishing given to an inner side surface of the external cylinder for the probe equipped in the cryosurgical apparatus.
[Fig. 5] Fig. 5 is a partial perspective view showing a barrier member arranged in a vacuum layer in the external cylinder for the probe equipped in the cryosurgical apparatus.

### [Description of Embodiments]

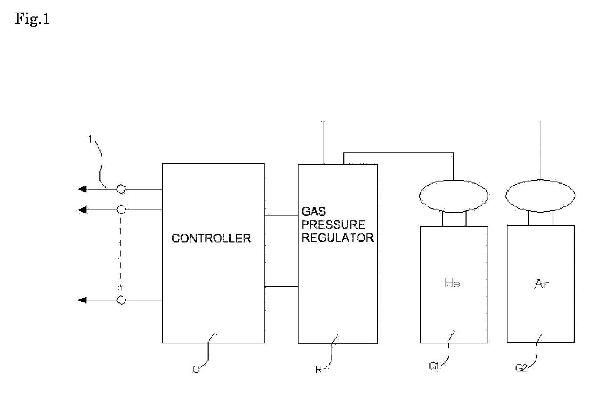

Fig. 1 is a diagram showing a basic configuration of a cryosurgical apparatus which employs a therapeutic-device unit according to the present invention. A reference 1 shows a therapeutic-device unit according to the present invention and, as described later, has a distal end made to puncture a lesion so as to penetrate therethrough, in a state of which a predetermined-type high-pressure gas is supplied to the unit. The therapeutic-device unit 1 is connected with gas supply sources G1 and G2 via a gas pressure regulator R which regulates the pressure of the predetermined-type high-pressure gas and a controller C which controls the gas pressure regulator R and controls an amount of gas being supplied and switching of gases being supplied.

The gas supply source G1 is a liquid gas cylinder charged with a freezing gas (in the present embodiment, helium gas), which is to be supplied to the therapeutic probe unit 1, whilst the gas supply source G2 is a liquid gas cylinder loaded with a thawing gas (in the present embodiment, argon gas), which is to be supplied the therapeutic probe unit 1.

The freezing gas and the thawing gas are supplied alternately supplied to the therapeutic-device unit 1 from the gas supply source G1 and the gas supply source G2. Meanwhile the controller C controls both switching valves (not shown) for the gasses being supplied and opening and closing valves (not shown) of the gas supply sources G1 and G2. A pressure of the gas supplied to the switching valve is detected by a not-shown pressure detecting section, and sent as data to the controller C. Further, an amount of supply of the gas which is sent out from the switching valve and an amount of return of the gasses returning to the switching valve are detected by a not-shown flow detecting section, and sent as data to the controller C. The controller C controls the switching valve, opening and closing valves, and gas pressure regulator R to maintain the detected data at values which are previously set or provided. The control for these members can be instructed by an operator, such as a practitioner in medicine, who instructs a man-machine interface including screens such as a touch panel.

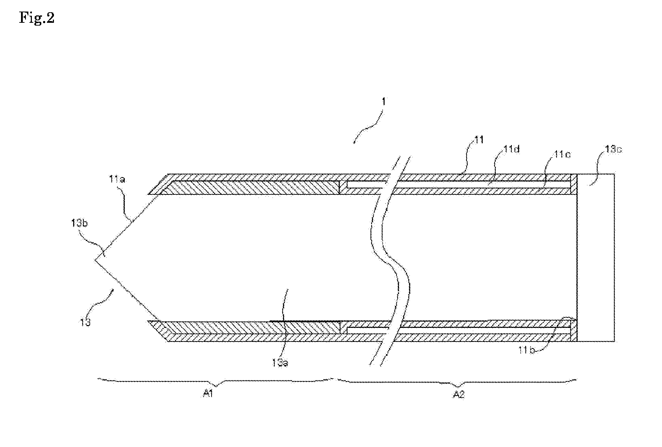

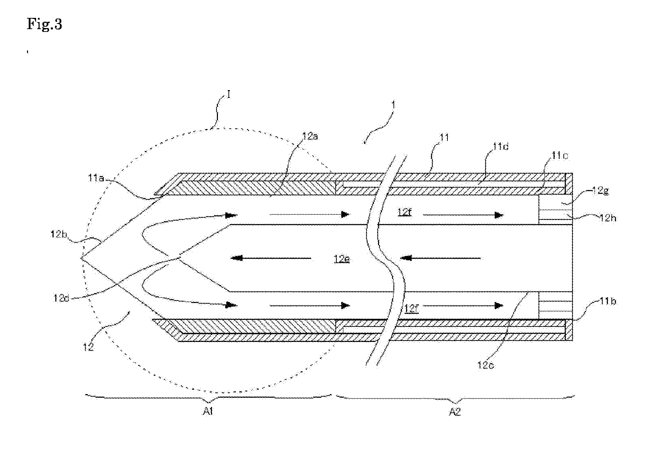

Fig. 2 shows a state where a guide needle is inserted through an external cylinder according to the present invention, and Fig. 3 shows a state where a probe is inserted through the external cylinder according to the present invention.

The therapeutic-device unit 1 is provided with, at least, a probe 12 to which the gasses are supplied and an external cylinder 11 through which the probe 12 is inserted. The external cylinder 11 is composed of, for example a stainless-steel cylindrical tube with no bottoms. The external cylinder 11 has a longitudinal direction and an opening end 11a located on the distal end side thereof in the longitudinal direction. The opening end 11a is formed in a taper-shaped end whose outer diameter is gradually reduced and whose shape is sharpened such that the tip end can be made to puncture a lesion. The opening end 11a has an inner diameter squeezed so that the opening end is circumscribed to a halfway position on sharpened distal end portions 12b and 13b of the probe 12 and the guide needle 13, which will be described later. The distal end portions are therefore supported by the opening end so that body portions 12a and 13a of the probe and the guide needle, which are larger in diameters than the distal end portions 12b and 13b, are not allowed to pass through the opening end 11a. Meanwhile the external cylinder 11 has a further opening end 11b formed on a base end side thereof in the longitudinal direction, and the opening end 11b has an outer diameter which makes it possible that the opening end 11b is closed by a tap member 12g of the probe 12, which will be described later. The sizes of the external cylinder 11 will not be limited to particular values as long as those sizes do not go beyond the gist of the present invention, and, as an approximate example, those sizes are set to be a whole length of 150 mm, an outer diameter ϕ of 3mm, and a tube wall thickness of 0.3 mm.

In the external cylinder 11, there is formed an adiabatic section 11c which extends from the base end side to a given positon in the longitudinal direction and which extends along the whole inner circumference. In the present embodiment, the adiabatic section 11c is formed by a vacuum layer 11d produced in an inner side portion and located in a closed space between the inner side portion and the outer side portion of the external cylinder 11. The closed space may be loaded with an adiabatic material as long as the adiabatic function is available. Alternatively, in addition to the closed space formed in the inner side portion of the external cylinder 11, it is possible to produce the closed space by covering the outer side portion of the external cylinder 11 on the whole circumference (not shown). In the external cylinder 11, there is thus provided a remaining end-side section with which the adiabatic section 11c is not formed and which is located close to the distal end in the longitudinal direction. As will de described later, ice balls are formed on the outer surface of the end-side section. Practically, the external cylinder 11 is provided to include an ice-ball formation range A1 and an adiabatic range A2 in the longitudinal direction. Additionally, the sizes of both of the ice-ball formation range A1 and the adiabatic range A2 can be decided adequately depending on applications. For example, when the ice balls are formed fully in a range of approx. 40 mm from the tip end of the external cylinder 11, the length of the adiabatic range A2 may be 110 mm or thereabouts. Further, the adiabatic section 11c has a thickness which is set depending on dimensional relationships with the outer diameter of the probe 12 described later. For example, in cases where the vacuum layer is formed with a width of approximately 0.1 mm, the adiabatic wall may be a thickness of approximately 0.1 mm.

The guide needle 13 is a puncture device which used to guide the external cylinder 11 to a lesion before a treatment performed using the therapeutic-device unit 1. As described before, the guide needle 13 has the cylindrical body portion 13a, the distal end portion 13b formed by sharpening the one end, and a flange portion 13b formed at the other end so as to have a diameter larger than the outer diameter of the base-side opening end 11b.

The probe 12 has the body portion 12a which is a hollow and cylindrical tool, the distal end portion 12b formed by sharpening the distal end of the body portion 12a. Inside the body portion 12a, an outward pipe 12c is inserted in such a manner that the outward pipe is approximately coaxially to the body portion 12a so that there is no contact between both the body portion and the outward path. The outward pipe 12c supplies the high-pressure gases (the freezing and thawing gasses) towards the distal end portion 12b. The distal end of the outward pipe 12c has an emission hole 12d so that the supplied high-pressure gas is emitted from the distal end of the probe 12. The probe 12 has an outward path 12e provided within the internal space of the outward pipe 12c and a return path 12f provided between the body portion 12a and the outward pipe 12c. The type of a material composing the probe 12 is not limited to a specific one, but by way of example, the probe may be a stainless-steel tube in the same way as the external cylinder 11. Further, since the probe is inserted and loaded through the external cylinder 11, the outer diameter ϕ may be 2.4 mm or thereabout, as an example.

Procedures of a treatment using the therapeutic-device unit 1 will now be explained. The guide needle 13 is inserted into the external cylinder 11 from its base-side opening end 11b such that the distal end portion 13b of the guide needle 13 is exposed from the tip-side opening end 11a of the external cylinder 11. The exposed distal end portion 13b of the guide needle 13 is percutaneously inserted toward a lesion which is a target being treated. With the guide needle 12 made to puncture the lesion, the external cylinder 11 is delivered in the puncture direction which is set coaxially to the guide needle 13. The process of puncture of both the guide needle 13 and the external cylinder 11 is repeated until they reach the lesion.

Under a known CT monitoring condition, the guide needle 13 is pulled out from the external cylinder 11 and it is confirmed that the external cylinder 11 has reached the lesion. After the confirmation that the external cylinder has reached the lesion, the probe 12 is inserted into the external cylinder 11 from its base-side opening end 11b. Distilled water or saline is injected in the external cylinder 11 before the puncture. Hence, this injection discharges air from the inside of the external cylinder 11 and produces a water screen between the external cylinder 11 and the probe 12. This production makes it possible to reduce friction caused therebetween, thus enabling the probe 12 to be inserted in a smoother manner.

In a state where the probe 12 is made to puncture the lesion, when the controller C described with Fig. 1 is instructed to act in a cryosurgery mode, the helium gas is supplied to the outward pipe 12c of the probe 12 from the gas supply source G1. The helium gas is given a predetermined pressure, which results in that the helium gas passes through the outward path 12e to reach the emission hole 12d and is emitted into the inside of the distal end portion 12b. The emitted helium gas expands rapidly therein, resulting in that Joule-Thompson effect makes both the distal end portion 12b of the probe 12 and the portion near the distal end of the external cylinder 11 (, which is the ice-ball formation range A1) cool down to an ultralow temperature, thereby freezing the lesion.

The frozen lesion tissue produces ice balls I containing the ice-ball formation range A1 of the external cylinder 11, but the adiabatic range A2 is not subjected to formation of ice balls and frost formation or ice accretion. Accordingly normal cells and tissue which are present close to the adiabatic range A2 will not be damaged by the ultralow temperature.

The helium gas is continuously supplied and emitted into the distal end portion 12b from the emission hole 12, whereby the emitted gas is forced to be delivered toward the return path 12f, with the delivered gas sent into the return path. The helium gas passing through the return path 12f is subjected to an adiabatic effect by the adiabatic section 11c, which results in cooling down the helium gas passing through the outward gas 12e so that the heat exchange action is accelerated, thus being effective use of the energy. The helium gas which has passed through the return path 12f is discharged into the atmosphere from a discharge hole 12h formed at the tap member 12g via not-shown discharge means.

The controller C is configured such that the controller is brought into a thawing treatment mode responsively to an elapse of a previously programed duration of supply of the helium gas. The thawing treatment mode switches the switching valves, whereby the current gas supply source is switched to the gas supply source G2 which supplies an argon gas, and the argon gas is then supplied through the outward pipe 12c of the probe 12. The argon gas has also a given gas pressure which makes the argon gas reach the emission hole 12d after passage through the outward path 12e. The argon gas is emitted into the distal end portion 12d of the probe 12, where the gas expands rapidly so that Joule-Thompson effect enables the distal end portion 12b of the probe 12 and the distal end range of the external cylinder 11 to be heated. Hence, the frozen lesion is thawed. In addition, the argon gas delivered through the return path 12e accelerates the heat exchange action, which is the same as that of the helium gas. The argon gas is also discharged via the discharge hole 12h, which is the same method as that of the helium gas.

The foregoing gas supply procedures are repeated at constant periods, with the result only the cells of the lesion become necrotic due to Joule-Thompson effect.

As described, in the conventional therapeutic-device unit, the heat exchange is performed in the heat exchanger provided in the outward pipe. In this case, the heat exchanger is obliged to be provided in a narrow space, thus making the structure complex and thus raising manufacturing costs. In this respect, the present invention overcomes the conventional difficulties such that arranging the simplified-structure adiabatic section 11c at the external cylinder 11 makes it possible to have the same actions as the conventional heat exchange action, which suppresses the manufacturing costs. Additionally, since the external cylinder 11 has the outer surface at which the adiabatic section 11c is provided, this limited-range outer surface does not undergo influence of the heat exchange action caused inside the cylinder, which prevents frost and/or ice from being formed and/or accreted, which realizes higher-accuracy treatment. Still additionally, the structure can be simplified as described, with the result that the inner volume of the return path 12f can be raised, reducing the discharge pressure, resulting in that the Joule-Thompson effect is effected more strongly than the conventional case.

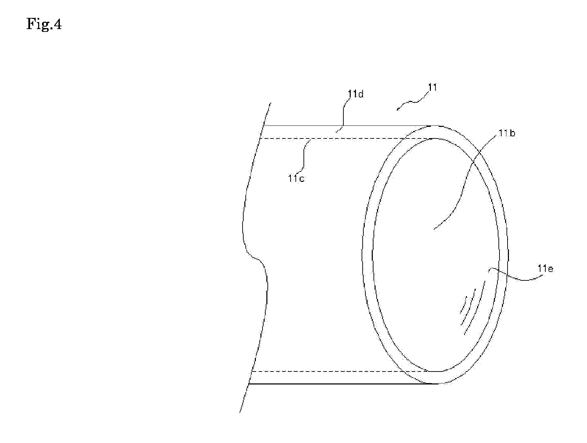

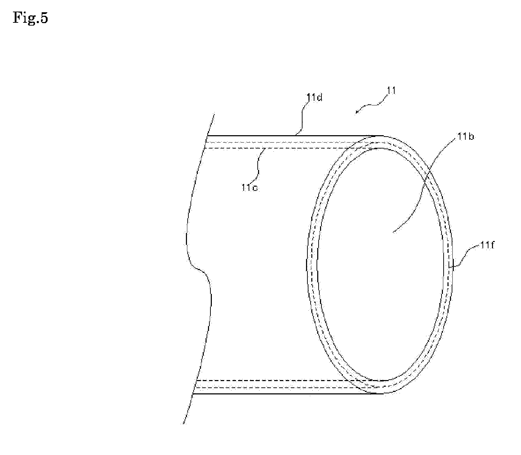

With reference to Figs. 4 and 5, a practical embodiment of the external cylinder 11 according to the present invention will now be described. In this description, the common components to those in Figs. 1, 2 and 3 are given the same reference numbers for omission of redundant explanations. Fig. 4 shows a partial perspective view of the external cylinder 11, in which the adiabatic section 11c has a surface 11e arranged in the wall of the external cylinder 11 via the vacuum layer 11d and specular finishing is applied to the surfaced 11e. Fig. 5 shows a partial perspective view, in which the vacuum layer 11d is charged with a barrier member 11f which is composed of beaten copper materials for example. In the structures shown in Figs. 4 and 5, radiation heat generated by the foregoing heat exchange action in the probe 12 is reflected to confine the heat energy more effectively within the probe 12, which prevents the heat from being transmitted easily to the outside of the external cylinder 11.

### [Reference Signs List]

- 1: therapeutic-device unit
- 11: external cylinder
- 11c: adiabatic section
- 11d: vacuum layer
- 12: probe
- 13: guide needle
- C: controller
- R: gas pressure regulator
- G1, G2: gas supply source
- A1: gas supply sources
- A2: adiabatic range

## Claims

1. An external cylinder for a probe used in a cryosurgical apparatus, wherein the external cylinder comprises a hollow inner space in which the probe for the cryosurgical apparatus is loaded, the probe comprising a distal end portion which is able to puncture a lesion located inside an object, the probe emitting a freezing gas and a thawing gas alternately to each other, **characterized in that**
the external cylinder comprises:
a given range in which the freezing gas enables an ice ball to be formed on an outer circumference including the distal end portion; and
adiabatic means arranged in a range other than the given range so as to prevent heat from being exchanged between the inner space and outside.

2. The external cylinder for the probe used in the cryosurgical apparatus according to claim 1, **characterized in that**
the adiabatic means comprises at least one of a closed space internally formed along a whole inner circumference of the external cylinder or a closed space formed to wholly cover an outer circumferential surface of the external cylinder.

3. The external cylinder for the probe used in the cryosurgical apparatus according to claim 2, **characterized in that**
the adiabatic means is a vacuum layer provided as either of the closed spaces, and
the adiabatic means comprises reflection means formed by applying specular finishing to a surface provided by the closed space.

4. The external cylinder for the probe used in the cryosurgical apparatus according to claim 2, **characterized in that**
the adiabatic means is a vacuum layer provided as either of the closed spaces, and
the adiabatic means comprises reflection means arranged in the closed space, the reflection means being composed of a barrier member.

5. The external cylinder for the probe used in the cryosurgical apparatus according to claim 2, **characterized in that** the adiabatic means is composed of either of the closed spaces, with which an adiabatic material is loaded.

6. A therapeutic-device unit, **characterized in that** the therapeutic-device unit comprises, at least, the probe equipped in the cryosurgical apparatus and the external cylinder for the probe equipped in the cryosurgical apparatus, which are according to any one of claims 1 to 3.
